Europäisches Patentamt

European Patent Office

Office européen des brevets

(19)

(11) **EP 1 584 612 A1**

(12) **EUROPEAN PATENT APPLICATION**
published in accordance with Art. 158(3) EPC

(43) Date of publication:
**12.10.2005 Bulletin 2005/41**

(51) Int Cl.⁷: **C07C 5/03**, C07C 15/085,
C07B 61/00

(21) Application number: **03780869.8**

(22) Date of filing: **18.12.2003**

(86) International application number:
**PCT/JP2003/016212**

(87) International publication number:
**WO 2004/063130 (29.07.2004 Gazette 2004/31)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **16.01.2003 JP 2003008049**

(71) Applicant: **Sumitomo Chemical Company,
Limited**
**Tokyo 104-8260 (JP)**

(72) Inventors:
• **OKU, Noriaki**
  **Ichihara-shi, Chiba 290-0035 (JP)**
• **KATAO, Masaaki**
  **Ichihara-shi, Chiba 290-0023 (JP)**

(74) Representative: **Lips, Hendrik Jan George**
**HAAGSCH OCTROOIBUREAU**
**Breitnerlaan 146**
**2596 HG Den Haag (NL)**

(54) **METHOD FOR HYDROGENATION OF OLEFIN**

(57)    A process for hydrogenating an_olefin, which comprises upwardly passing a liquid containing an olefin and a gas containing hydrogen through a packed bed of a solid hydrogenation catalyst, wherein the superficial velocity of the gas is 3.0 cm/sec or more.

EP 1 584 612 A1

**Description**

**TECHNICAL FIELD**

The present invention relates to a process for hydrogenating an olefin.

**BACKGROUND ART**

As a process for hydrogenating an olefin, by which a liquid containing an olefin and a gas containing hydrogen are passed upwardly through a packed bed of a solid hydrogenation catalyst, for example, U.S.Patent 3,127,452 discloses a technique in which $\alpha$-methyl styrene and hydrogen are supplied to a catalyst bed under an upward flow to carry out hydrogenation. However, conventional processes were not necessarily satisfied from the view point of production of cumene at low cost.

**DISCLOSURE OF THE INVENTION**

The present invention provides a process for hydrogenating an olefin, having an excellent effect on which the reaction rate per unit of a catalyst is high.

That is, the present invention relates to a process for hydrogenating an olefin, which comprises passing upwardly a liquid containing an olefin and a gas containing hydrogen through a packed bed of a solid hydrogenation catalyst, wherein the superficial velocity of the gas is 3.0 cm/sec or more.

**MODE FOR CARRYING OUT THE INVENTION**

As a solid hydrogenation catalyst used for hydrogenation of an olefin, for example, a metal oxide containing at least one of CuO, $Cr_2O_3$, ZnO , $FeO_3$, $Al_2O_3$, $La_2O_3$, $Sm_2O_3$, $CeO_2$, $ZrO_2$, $TiO_2$, $SiO_2$, $MnO_2$, $CO_2O_3$, NiO, BaO, CaO and MgO; and a noble metal catalyst containing Pd, Rh, Pt or Ru, can be listed. As the solid hydrogenation catalyst, a carrier may be used or not. As the carrier, there can be listed metal oxides or compound oxides thereof such as silica, alumina, titania, zirconia, magnesia and silica-alumina; and bentonite, montmorillonite, diatomaceous earth, acidic white clay, active carbon, ceramics and the like.

In addition, as a shape of the catalyst, sphere and cylindrical form are illustrated. The catalyst is usually 0.5 to 10 mm in size, and when the size is too small, a pressure loss increases and leads to uneconomical, on the other hand, when too large, it is not preferable because a catalyst activity decreases and a reaction fluid flows non-uniformly.

The hydrogenation of an olefin is carried out using a reactor in which the solid hydrogenation catalyst is packed. The reaction temperature is usually 20 to 500°C, preferably 40 to 350°C, and the reaction pressure is usually 0.1 to 20 MPa, preferably 0.1 to 10 MPa.

The used amount of the catalyst is 0.01 to 50 $hr^{-1}$ in terms of a space velocity of the liquid containing the olefin supplied, and a liquid containing a fresh olefin as a raw material may be diluted with an appropriate solvent or with a liquid obtained by recycling a part of the reaction mixture liquid after hydrogenation. The amount of hydrogen is usually 1.0 to 30 times by mole per an olefin to be supplied, further, hydrogen may be diluted with a gas which does not react with the olefin supplied, and extra hydrogen may be recycled.

As the olefin, compounds having a double bond or triple bond such as a styrene can be listed. The styrene includes styrene, $\alpha$-methyl styrene and the like.

In the present invention, a liquid containing an olefin and a gas containing hydrogen are passed upwardly (upflow) through a packed bed of a solid hydrogenation catalyst. As reasons why the upflow is applied, a runaway of the reaction according to difficulty of control of the reaction temperature caused by generation of hot spots, can be suppressed because the liquid can be uniformly flowed in the packed bed without localization.

The utmost characteristic of the present invention is to control a superficial velocity of the gas to 3.0 cm/sec or higher, preferably 3.5cm/sec or higher. When the superficial velocity of the gas is lower than 3.0 cm/sec, the apparent reaction rate lowers, namely the reacted amount per unit of the packed bed lowers because a rate of which hydrogen dissolves in the liquid, controls, due to a fast rate of olefin hydrogenation, the rate of the reaction. In addition, the lowering of the hydrogenation rate of the olefin leads to lowering of the yield through a tar formation reaction caused by formation of an olefin dimer and olefin polymer. The superficial velocity of the gas is preferably 10 cm/sec or lower. When the superficial velocity of the gas is higher than 10 cm/sec, a pressure loss of the packed bed may increase because wear and tear powder of the catalyst is formed by friction among catalysts.

The superficial velocity of a gas is determined by the following equation (1):

(Superficial velocity of gas) = (Real volume velocity of gas) /

(Cross-sectional area of reactor)                    (1)

**EXAMPLE**

Next, the present invention is explained by Examples.

Example 1

A catalyst (12 cc) containing copper was packed into a reactor having an inner diameter of 4 mm, a liquid containing α-methyl styrene (AMS) of 21% by weight was supplied at a rate of 48 g/min and hydrogen of 1.5 times by mole per mole of AMS fed was fed to the reactor. The reaction was carried out under a pressure of 1.0 MPa at a temperature of 200°C. At this time, the superficial velocity of the gas was 7 cm/sec, and the reaction amount of α-methyl styrene per catalyst was 49 kmol/m$^3$ catalyst/hr.

Example 2

A catalyst (2.2 cc) containing palladium was packed into a reactor having an inner diameter of 4 mm, a liquid containing α-methyl styrene (AMS) of 18% by weight was fed at a rate of 3.3 g/min and hydrogen of 1.5 times by mole per mole of AMS fed was fed to the reactor. The reaction was carried out under a pressure of 1.0 MPa at a temperature of 180°C. At this time, the superficial velocity of gas was 6. 5 cm/sec, and the reaction amount of α-methyl styrene per catalyst was 71 kmol/m3 catalyst/hr.

Comparative Example 1

This experiment was carried out under the same conditions as in Example 1 except that the raw material was fed at a rate of 1.6 g/min and the reaction was carried out at a temperature of 210°C under a pressure of 1.4 MPa. At this time, the superficial velocity of gas was 2.7 cm/sec, and the reaction amount of α-methyl styrene per catalyst was 14 kmol/m$^3$ catalyst/hr.

Comparative Example 2

This experiment was carried out under the same conditions as in Example 2 except that the raw material was fed at a rate of 1.6 g/min. At this time, the superficial velocity of gas was 2.8 cm/sec, and the reaction amount of α-methyl styrene per catalyst was 42 kmol/m$^3$ catalyst/hr.

**INDUSTRIAL APPLICABILITY**

As described above, according to the present invention, there is provided a process for hydrogenating an olefin, under which a liquid containing the olefin and a gas containing hydrogen are passed upwardly through a packed bed of a solid hydrogenation catalyst, wherein the process has an excellent effect that the reaction rate per unit catalyst is high.

**Claims**

1.   A process for hydrogenating an olefin, which comprises passing upwardly a liquid containing an olefin and a gas containing hydrogen through a packed bed of a solid hydrogenation catalyst, wherein the superficial velocity of the gas is 3.0 cm/sec or more.

2.   The process according to claim 1, wherein the olefin is a styrene.

3.   The process according to claim 1 or 2, wherein the superficial velocity of the gas is 3.5 to 10 cm/sec.

**EP 1 584 612 A1**

<table>
<tr><td colspan="2" align="center"><b>INTERNATIONAL SEARCH REPORT</b></td><td colspan="2">International application No.<br>PCT/JP03/16212</td></tr>
</table>

**A. CLASSIFICATION OF SUBJECT MATTER**
Int.Cl$^7$ C07C5/03, 15/085, C07B61/00

According to International Patent Classification (IPC) or to both national classification and IPC

**B. FIELDS SEARCHED**

Minimum documentation searched (classification system followed by classification symbols)
Int.Cl$^7$ C07C5/03, 15/085, C07B61/00

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | US 3127452 A (Societa Italiana Resine),<br>31 March, 1964 (31.03.64),<br>Example 2; Claims<br>(Family: none) | 1-3<br>1-3 |
| X<br>Y | JP 56-140933 A (Sumitomo Chemical Co., Ltd.),<br>04 November, 1981 (04.11.81),<br>Claims; examples<br>(Family: none) | 1-3<br>1-3 |
| Y | JP 6-32747 A (Sumitomo Chemical Co., Ltd.),<br>08 February, 1994 (08.02.94),<br>Claims; examples<br>(Family: none) | 1-3 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>15 March, 2004 (15.03.04) | Date of mailing of the international search report<br>06 April, 2004 (06.04.04) |
|---|---|
| Name and mailing address of the ISA/<br>Japanese Patent Office | Authorized officer |
| Facsimile No. | Telephone No. |

Form PCT/ISA/210 (second sheet) (July 1998)

4

**EP 1 584 612 A1**

<table>
<tr><td colspan="3" align="center"><strong>INTERNATIONAL SEARCH REPORT</strong></td><td>International application No.<br>PCT/JP03/16212</td></tr>
</table>

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | GB 1555270 A (ENGELHARD MINERALS & CHEMICALS CORP.),<br>07 November, 1979 (07.11.79),<br>Examples; Claims<br>& JP 53-82704 A        & DE 2758274 A1<br>& FR 2376100 A1        & GB 1555270 A | 1-3 |
| Y | US 4257877 A (Engelhard Minerals & Chemicals Corp.),<br>24 March, 1981 (24.03.81),<br>Examples; Claims<br>& DE 2758318 A1        & FR 2376101 A1<br>& GB 1543880 A        & JP 53-82703 A | 1-3 |

Form PCT/ISA/210 (continuation of second sheet) (July 1998)

5